Europäisches Patentamt

European Patent Office

Office européen des brevets

(11) Veröffentlichungsnummer: **0 231 300**
**B1**

(12) **EUROPÄISCHE PATENTSCHRIFT**

(45) Veröffentlichungstag der Patentschrift:
24.05.89

(51) Int. Cl.⁴: **B 01 L 3/00, G 01 N 33/50**

(21) Anmeldenummer: 86904776.1

(22) Anmeldetag: 06.08.86

(86) Internationale Anmeldenummer:
PCT/DE 86/00320

(87) Internationale Veröffentlichungsnummer:
WO 87/00769 (12.02.87 Gazette 87/4)

(54) Verfahren zur Hemmung der Reduktion des Glucose-Gehalts im Blut.

(30) Priorität: 08.08.85 DE 3528730
09.09.85 DE 3532477
07.11.85 DE 3539784

(43) Veröffentlichungstag der Anmeldung:
12.08.87 Patentblatt 87/33

(45) Bekanntmachung des Hinweises auf die Patenterteilung:
24.05.89 Patentblatt 89/21

(84) Benannte Vertragsstaaten:
AT BE CH DE FR GB IT LI NL SE

(73) Patentinhaber: LISS, Eberhard, Schütte-Lanz-Str. 94 a,
D-1000 Berlin 45 (DE)
Patentinhaber: LISS, Inge, Schütte-Lanz-Str. 94 a,
D-1000 Berlin 45 (DE)

(72) Erfinder: LISS, Eberhard, Schütte-Lanz-Str. 94 a,
D-1000 Berlin 45 (DE)
Erfinder: LISS, Inge, Schütte-Lanz-Str. 94 a,
D-1000 Berlin 45 (DE)

(74) Vertreter: Patentanwaltsbüro Cohausz & Florack,
Postfach 14 01 20, D-4000 Düsseldorf 1 (DE)

(56) Entgegenhaltungen:
US-A- 3 925 153
US-A- 4 054 488

Clinical Chemistry, Band 26, Nr. 8, Juli 1980, Winston,
Salem (US). E.A. Robertson et al.: "Serum collected
with lithium iodacetate is unsuitable for electrolyte and
lactate dehydrogenase measurements", Seiten
1228-1229
Chemical Abstracts, Band 66, Nr. 7, 13. Februar 1967,
Columbus, Ohio (US). E. Beutler et al.: "Blood
preservation relative capacities of hexoses, hexitols,
and ethanol to maintain red cell ATP levels during
storage", Seite 2546, Zusammenfassung Nr. 27053u.
Clinical Chemistry, Band 25, Nr. 4, April 1979, Winston,

(56) Entgegenhaltungen: (Fortsetzung)
Salem (US). S. Meltes et al.: "Preservation, distribution
and assay of glucose in blood, with special reference to
the newborn", Seiten 531-534.
Chemical Abstracts, Band 89, Nr. 17, 23. Oktober 1978,
Columbus, Ohio (US). R.B. Dawson et al.: "Blood
preservation. XXVII. Fructose and mannose maintain
ATP and 2,3-DPG", Seite 387, Zusammenfassung Nr.
144188c.

## Beschreibung

Die Erfindung betrifft ein Verfahren zur Hemmung der Reduktion des Glucosegehalts im entnommenen Blut mittels bestimmter Zusätze. Die Zusätze sollen gewährleisten, dass der Glucosegehalt in dem entnommenen Blut (Blutzucker) so wenig wie möglich oder gar nicht absinkt.

Im Zusammenhang mit der Entnahme und Aufbewahrung von Blut hat sich gezeigt, dass der Glucosegehalt in dem entnommenen Blut fortlaufend absinkt (Testfibel Gluco-quant[R] Glucose, Firmenschrift Boehringer, Mannheim, 1984, S. 23), so dass schon nach einstündigem Aufbewahren bei Zimmertemperatur ein Verlust von mehr als 10% auftreten kann (Thomas, Labor und Diagnose, 2. Auflage, Die Medizinische Verlagsgesellschaft, Marburg/Lahn, 1984, S. 118). Der Verlust kommt dadurch zustande, dass durch den Stoffwechsel der noch lebenden Zellen des Blutes auch nach der Blutentnahme die Glucose verbraucht wird. Um diesen Veränderungen in der Zusammensetzung des Blutes für Zwecke der Laboratoriumsdiagnostik zu begegnen, werden dem Blut sofort nach der Entnahme geeignete Substanzen zugefügt, welche den Stoffwechsel der Blutzellen vergiften, um die Zellen gezielt daran zu hindern, weiterhin Glucose zu verbrauchen. Dieses Verfahren ist weit verbreitet. Es werden im Handel Entnahmespritzen und Aufbewahrungsbehälter angeboten, in denen die vergiftenden Substanzen bereits enthalten sind. Verwendung finden vor allem Monojodacetat (Zeitschrift „Clinical Chemistry", Band 21 [1975] S. 1810; „Clinical Chemistry", Band 24 [1978] S. 998) und Fluoride, z. B. NaF und KF (Thomas, Labor und Diagnose, 2. Aufl., Die Medizinische Verlagsgesellschaft, Marburg/Lahn, 1984, S. 119; R. Richterich und J.P. Colombo: Klinische Chemie, 4. Auflage. S. Karger, Basel, München, Paris, London, New York, Sydney, 1978, S. 307; W. Rick: Klinische Chemie und Mikroskopie, 3. Auflage, Springer Verlag, Berlin, Heidelberg, New York, 1974, S. 177).

Die genannten Substanzen, Fluoride bzw. Salze der Monojodessigsäure, verringern zwar den Schwund der Glucose durch Vergiftung der Verbrauchsvorgänge, jedoch führt

1. die Behandlung des Blutes mit diesen Substanzen zu einer Schädigung der Blutzellen, und es ist

2. der erzielte Effekt in quantitativer Hinsicht unbefriedigend.

Zu 1. Die Schädigung der Blutzellen äussert sich u. a. in den Veränderungen der Permeabilität, so dass Stoffe aus dem Zellinnern in die Blutflüssigkeit abfliessen. Dadurch ändert sich die Konzentration dieser Stoffe in der Blutflüssigkeit, so dass deren analytische Erfassung zu Ergebnissen führt, die nicht den Zustand im frisch entnommenen Blut repräsentieren.

Dabei handelt es sich um diagnostisch besonders bedeutsame Substanzen, wie Kalium und Enzyme (Zeitschrift „Clinical Chemistry", Band 26 [1980] S. 1228). Weiterhin haben die vergiftenden Substanzen in einigen Fällen Einfluss auf die Analysenmethoden, so dass auf diese Weise ebenfalls falsche Untersuchungsergebnisse resultieren können. So wird z. B. in Gegenwart von Fluoriden die Erfassung des Harnstoffs gestört (Zeitschrift „Clinical Chemistry", Band 20 [1974] S. 876) und in Gegenwart von Monojodacetat die Erfassung der Creatinkinase (Zeitschrift „Clinical Chemistry", Band 26 [1980] S. 1228). Wegen der erforderlichen relativ hohen molaren Konzentrationen an Fluoriden resultieren allein aus osmotischen Gründen eine Reihe zusätzlicher Veränderungen. Ausserdem wird im Falle der Verwendung von Fluoriden das Gerinnen des Blutes verhindert, so dass keine Wahlmöglichkeit mehr besteht, Untersuchungen sowohl im Plasma wie im Serum durchzuführen.

Diese Probleme haben an vielen Orten zu dem unwirtschaftlichen Ergebnis geführt, dass für die Glucosebestimmung eine gesonderte Blutprobe gewonnen werden muss, während viele andere Parameter aus einer einzigen Probe bestimmt werden können. Dies ist besonders nachteilig, wenn mit modernen Grossgeräten viele Parameter gleichzeitig analysiert werden sollen. Bei der jüngst erfolgten Beschreibung des Mehrfachanalysensystems (Zeitschrift „Labormedizin", Heft 1/1985, S. 17) fehlt daher der Parameter Glucose.

Zu 2. Durch die genannten Verbindungen wird der Verbrauch der Glucose nur unvollständig verhindert, so dass z. B. trotz Verwendung von Fluoriden 2 Stunden nach der Blutentnahme nur 88,1-89,9% (Zeitschrift „Clinical Chemistry", Band 28 [1982] S. 190) bzw. nur 91,9% (Zeitschrift „The Lancet", Jahrgang 1984, S. 1165 und Jahrgang 1985, S. 704) der ursprünglich vorhandenen Glucose erfasst werden. Auch Mischungen aus Fluoriden und Salzen der Monojodessigsäure führen zu unbefriedigenden Resultaten (Zeitschrift „Clinical Chemistry", Band 25 [1979] S. 531).

Aus der US-PS 39 25 153 ist schliesslich ein Gemisch bekannt, welches aus Dihydroxyaceton und Nicotinamid besteht. Mit dessen Hilfe lässt sich Blut konservieren. Das Problem der Reduzierung des Glucosegehalts wird in der US-PS jedoch nicht angesprochen.

Die vorliegende Erfindung hat sich nunmehr die Aufgabe gestellt, ein Verfahren zur Hemmung der Reduktion des Glucosegehalts von entnommenem Blut unter Verwendung von Zusatzstoffen zu schaffen.

Gelöst wird diese Aufgabe dadurch, dass als Zusatzstoffe eine oder mehrere Verbindungen aus der Gruppe der Kohlenhydrate und ihrer Derivate in Mengen von jeweils mehr als 0,1 mg/ml Blut allein oder gemeinsam mit anderen Substanzen verwendet werden.

Die erfinderische Lösung besteht in der Anwendung eines bisher für das Problem der Glucosekonservierung in Blutproben nicht genutzten Prinzips. Nach diesem neuen Prinzip wird der Verbrauch der Glucose dadurch reduziert, dass den Blutproben der Glucose chemisch verwandte Substanzen zugefügt werden, welche im Stoffwechsel der Blutzellen an die Stelle der Glucose treten und diese damit von ihren Wirkorten verdrängen. Dadurch wird die Glucose langsamer oder gar nicht mehr verbraucht. Für die Anwendung kommen alle Substanzen aus der Gruppe der Kohlenhydrate und ihrer Derivate in Betracht, die im Stoffwechsel der Blutzellen den Platz der Glucose einnehmen und diese damit ersetzen oder verdrängen können.

Unter Ausnutzung dieser Möglichkeiten ergeben sich zwei getrennte Anwendungsbereiche:

1. Anders als bei Verwendung von Stoffwechselgiften werden bei Verwendung der erfindungsgemäss vorgeschlagenen Substanzen die Blutzellen nicht geschädigt. Die Konzentrationen der in der Blutflüssigkeit ausser Glucose vorliegenden, labordiagnostisch relevanten Substanzen werden daher nicht durch den Zustrom von Substanzen aus geschädigten oder veränderten Blutzellen verfälscht. Die Konservierung der Glucose im Vollblut mit den erfindungsgemäss vorgeschlagenen Substanzen ermöglicht somit erstmals die Analyse von konservierter Glucose neben anderen Parametern aus der gleichen Blutprobe.

Im Durchschnitt wird die Glucose durch die erfindungsgemäss vorgeschlagenen Substanzen mindestens ebensogut konserviert wie bei Verwendung der herkömmlichen Stoffwechselgifte.

2. Sowohl die konventionelle Anwendung von Stoffwechselgiften wie auch die jetzt erfindungsgemäss vorgeschlagene Anwendung von Substanzen, welche die Glucose verdrängen, verhindern den Verbrauch der Glucose und damit die Verminderung ihrer Konzentration nicht vollständig. Wie bereits erwähnt, sinkt die Glucosekonzentration im Blut trotz Verwendung der herkömmlichen Konservierungsmittel ab, z. B. in zwei Stunden auf 89 bis 92% der Ausgangswerte. Weil aber die konventionelle und die erfindungsgemässe Konservierungsmethode auf unterschiedlichen Mechanismen beruhen, lassen sich durch gleichzeitige Anwendung beider Verfahren wesentlich bessere Konservierungseffekte erzielen als durch Anwendung entweder des konventionellen oder des erfindungsgemässen Verfahrens. So sind im Durchschnitt nach zweistündiger Aufbewahrung von Vollblut in Gegenwart beider Arten von Konservierungsmitteln noch 99% der ursprünglich vorhandenen Glucose nachweisbar. Weil bei Blut von Neugeborenen aus bestimmten Gründen die Konservierung mit Stoffwechselgiften wesentlich schlechter ist als bei Blut von Erwachsenen, ergeben sich in diesem Fall besonders auffällige Verbesserungen bei gleichzeitiger Anwendung beider Konservierungsmittel.

Für jeden der beiden Anwendungsbereiche wird lediglich eine bestimmte Ausführungsform von zahlreichen möglichen Ausführungsformen angegeben. Danach sind Behälter, die für die Aufnahme von 2 ml Vollblut geeignet sind, z. B. Kunststoffröhrchen

1. mit 4 mg d-Mannose zu beschicken bzw.

2. mit 4 mg d-Mannose und 5 mg Kaliumfluorid zu beschicken. Die Substanzen können in wässeriger Lösung eingebracht und anschliessend eingetrocknet werden. Dann ergeben sich keine Volumenänderungen des Blutes durch die zugegebenen Substanzen.

Die gewerbliche Verwertbarkeit der Erfindung ist offensichtlich, da seit längerer Zeit Behälter für Blutproben im Handel sind, die mit den konventionellen Konservierungsmitteln für Glucose beschickt sind.

## Patentansprüche

1. Verfahren zur Hemmung der Reduktion des Glucosegehaltes in entnommenem Blut unter Verwendung von Zusatzstoffen, dadurch gekennzeichnet, dass als Zusatzstoffe eine oder mehrere Verbindungen aus der Gruppe der Kohlenhydrate und ihrer Derivate in Mengen von jeweils mehr als 0,1 mg/ml Blut allein oder gemeinsam mit anderen Substanzen verwendet werden.

2. Verfahren gemäss Anspruch 1, dadurch gekennzeichnet, dass als Zusatzstoff eine oder mehrere zur Glucose isomere Substanzen in Mengen von mehr als 0,1 mg/ml Blut allein oder gemeinsam mit anderen Substanzen verwendet werden.

3. Verfahren gemäss Anspruch 1, dadurch gekennzeichnet, dass als Zusatzstoff d-Mannose oder ein Derivat der d-Mannose in Mengen von mehr als 0,1 mg/ml Blut allein oder gemeinsam mit anderen Substanzen verwendet wird.

4. Verfahren gemäss Anspruch 1, dadurch gekennzeichnet, dass ein Zusatz eingesetzt wird, der folgende Bestandteile enthält:

a) eine oder mehrere Verbindungen aus der Gruppe der Kohlenhydrate und ihrer Derivate in Mengen von jeweils mehr als 0,1 mg/ml Blut,

b) eine oder mehrere Verbindungen aus der Gruppe der Fluoride in Mengen von jeweils mehr als 0,1 mg/ml Blut

oder ein oder mehrere Salze der Monojodessigsäure in Mengen von jeweils mehr als 0,05 mg/ml Blut

oder sowohl Verbindungen aus der Gruppe der Fluoride als auch Salze der Monojodessigsäure, und

c) ggf. weitere Verbindungen.

5. Verfahren nach Anspruch 1, dadurch gekennzeichnet, dass ein Zusatz eingesetzt wird, der folgende Bestandteile enthält:

a) d-Mannose oder ein Derivat der d-Mannose in Mengen von jeweils mehr als 0,1 mg/ml Blut,

b) eine oder mehrere Verbindungen aus der Gruppe der Fluoride in Mengen von jeweils mehr als 0,1 mg/ml Blut

oder ein oder mehrere Salze der Monojodessigsäure in Mengen von jeweils mehr als 0,05 mg/ml Blut

oder sowohl Verbindungen aus der Gruppe der Fluoride als auch Salz der Monojodessigsäure, und

c) ggf. weitere Verbindungen.

## Claims

Method for inhibition of the reduction of the glucose-content in collected blood by use of additives,
characterized in that, as additive, one or a plurality of substances are used selected from the group of carbohydrate and derivatives thereof in amounts of more than 0.1 mg/ml blood alone or in combination with other substances.

2. Method in accordance with claim 1, characterized in that, as additive, one or a plurality of substances are used which are isomeric with respect to glucose in amounts of more than 0.1 mg/ml blood alone or in combination with other substances.

3. Method in accordance with claim 1, characterized in that, as additive, d-mannose or a derivative of the d-mannose is used in amounts of more than 0.1 mg/ml blood alone or in combination with other substances.

4. Method in accordance with claim 1, characterized in that the additive contains the following components:
a) one or a plurality of compounds from the group of the carbohydrates and the derivatives thereof in amounts of each more than 0.1 mg/ml blood,
b) one or a plurality of compounds from the group of the fluorides in amounts of each more than 0.1 mg/ml blood, or
one or a plurality of salts of the monoiodine acetic acid in amounts of each more than 0.05 mg/ml blood, or
as well as compounds from the group of the fluorides as well as salts from the monoiodine acetic acid,
c) optionally further compounds.

5. Method in accordance with claim 1, characterized in that the additive contains the following components:
a) d-mannose or a derivative of the d-mannose in amounts of each more than 0.1 mg/ml blood,
b) one or a plurality of compounds from the group of the fluorides in amounts of each more than 0.1 mg/ml blood, or
one or a plurality of salts of the monoiodine acetic acide in amounts of each more than 0.05 mg/ml blood, or
compounds from the group of the fluorides as well as salts of the monoiodine acetic acid,
c) optionally further compounds.

## Revendications

1. Procédé pour inhiber la réduction de la teneur en glucose de sang prélevé avec l'utilisation d'additifs, caractérisé par le fait qu'on utilise, comme additifs, un ou plusieurs composés du groupe des glucides et de leurs dérivés, en des quantités de plus de 0,1 mg/ml de sang à chaque fois, seul(s) ou conjointement avec d'autres substances.

2. Procédé selon la revendication 1, caractérisé par le fait qu'on utilise, comme additifs, une ou plusieurs substances isomères du glucose, en des quantités de plus de 0,1 mg/ml de sang, seule(s) ou conjointement avec d'autres substances.

3. Procédé selon la revendication 1, caractérisé par le fait qu'on utilise, comme additif, le d-mannose ou un dérivé du d-mannose, en des quantités de plus de 0,1 mg/ml de sang, seul ou conjointement avec d'autres substances.

4. Procédé selon la revendication 1, caractérisé par le fait qu'on emploie un additif qui renferme les constituants suivants:
a) un ou plusieurs composés du groupe des glucides et de leurs dérivés, en des quantités à chaque fois de plus de 0,1 mg/ml de sang,
b) un ou plusieurs composés du groupe des fluorures, en des quantités de plus de 0,1 mg/ml de sang à chaque fois, ou un ou plusieurs sels de l'acide mono-iodo-acétique, en des quantités de plus de 0,05 mg/ml de sang à chaque fois, ou non seulement des composés du groupe des fluorures mais aussi des sels de l'acide mono-iodo-acétique, et
c) le cas échéant, d'autres composés.

5. Procédé selon la revendication 1, caractérisé par le fait qu'on emploie un additif qui renferme les constituants suivants:
a) le d-mannose ou un dérivé du d-mannose en des quantités de plus de 0,1 mg/ml de sang à chaque fois,
b) un ou plusieurs composés du groupe des fluorures, en des quantités de plus de 0,1 mg/ml de sang à chaque fois, ou un ou plusieurs sels de l'acide mono-iodo-acétique, en des quantités de plus de 0,05 mg/ml de sang à chaque fois, ou non seulement des composés du groupe des fluorures mais aussi des sels de l'acide mono-iodo-acétique, et
c) le cas échéant, d'autres composés.